# EUROPEAN PATENT APPLICATION

(11) **EP 2 987 448 A1**
(43) Date of publication of application: **24.02.2016**
(21) Application number: 14784990.5
(22) Date of filing: 16.04.2014
(51) Int. Cl.: A61B 1/00, A61B 1/04, G02B 23/24, H04N 5/225, H04N 7/18

(54) **IMAGE CAPTURING DEVICE AND ELECTRONIC ENDOSCOPE**

(30) Priority: 18.04.2013 JP 2013087583; 18.04.2013 JP 2013087584
(71) Applicant: Olympus Corporation, Shibuya-ku Tokyo 151-0072 (JP)
(72) Inventor: IGARASHI Takatoshi, Tokyo 151-0072 (JP)
(74) Representative: Winter, Brandl, Fürniss, Hübner, Röss, Kaiser, Polte - Partnerschaft mbB
(86) International application number: PCT/JP2014/060818
(87) International publication number: WO 2014/171482

(57) **Abstract**

An image pickup apparatus 40 includes a laminated substrate 46 on which a plurality of electronic components 55 to 58 configuring a driving circuit of a solid-state image pickup device 44 are mounted and in which a plurality of conductor layers 76 to 78 and a plurality of vias 71 to 75 are formed, electronic component connection lands 61 and 62 provided on a surface of the laminated substrate 46, any one of the plurality of electronic components 55 to 58 being electrically connected to the electronic component connection lands 61 and 62, and a cable connection land 63 provided on the surface of the laminated substrate 46, a plurality of signal cables 48 being electrically connected to the cable connection land 63. At least one of the plurality of electronic components 55 to 58 is embedded in a position superimposed on the electronic component connection lands 61 and 62 or the cable connection land 63 on an inside of the laminated substrate 46.

## Description

### Technical Field

The present invention relates to an image pickup apparatus and an electronic endoscope in which the image pickup apparatus is provided.

### Background Art

As it is well known, endoscopes are widely used for observation, treatment, and the like for medical use in bodies (body cavities) of living organisms or inspection, repairing, and the like in plant facilities for industrial use. In particular, an endoscope for medical use can observe an examination target region in a body cavity without requiring dissection because an elongated insertion section is inserted into the body cavity. Curative treatment can be performed using a treatment instrument according to necessity. Therefore, the endoscope is widely used.

As such an endoscope, for example, there is an electronic endoscope in which an image pickup apparatus is disposed at a distal end portion of an insertion section disclosed in Japanese Patent Application Laid-Open Publication No. 2005-304876. In such a conventional electronic endoscope, a circuit board attached with electronic components such as a capacitor and an IC chip configuring a driving circuit and the like of a solid-state image pickup device is provided in the conventional image pickup apparatus incorporated at a distal end of the insertion section.

The image pickup apparatus used in the conventional electronic endoscope has structure in which the circuit board is adjacent to a rear side of the solid-state image pickup device and fixed to the solid-state image pickup device. Note that, in the conventional image pickup apparatus, terminals are provided on a rear end side of the circuit board and signal cables are soldered to these terminals.

Further, in the image pickup apparatus of the conventional electronic endoscope, cables connected to the circuit board are soldered to cable connection lands formed close to one another on the same surface of the circuit board.

However, in the image pickup apparatus incorporated in the conventional electronic endoscope, the electronic components and the cable connection terminals are formed on the same plane on the circuit board. Therefore, there is a problem in that a predetermined area is necessary for respectively connecting the electronic components and the signal cables and a reduction in size is limited.

Therefore, there is a problem in that a reduction in size of the distal end portion of the insertion section of the electronic endoscope incorporating the image pickup apparatus is also limited and a reduction in a diameter of the insertion section of the electronic endoscope is hindered.

Further, when an image signal cable and a synchronization signal cable are disposed adjacent to each other on the circuit board as in the electronic endoscope disclosed in Japanese Patent Application Laid-Open Publication No. 2005-304876, it is likely that an electronic magnetic wave generated from the synchronization signal cable interferes with an image signal transmitted by the image signal cable and noise occurs. When the noise occurs in the image signal, there is a problem in that an endoscopic image displayed on a monitor is disturbed and becomes unstable.

Note that, as disclosed in Japanese Patent Application Laid-Open Publication No. 2005-304876, distances among the cables can be increased by, for example, providing relay members on cable connection lands of the circuit board to provide level differences among connecting sections of the various signal cables or provide cable connection lands on front and rear surfaces of the board. However, there is still a problem in that a process for attaching the relay members is added and soldering on the front and rear surfaces of the board is troublesome.

Further, since the relay member is provided on the circuit board, the circuit board is increased in size and a reduction in size of the image pickup apparatus is limited. Therefore, there is a problem in that a reduction in size of the distal end portion of the insertion section of the electronic endoscope incorporating the image pickup apparatus is also limited and a reduction in a diameter of the insertion section of the electronic endoscope is hindered.

Therefore, the present invention has been devised in view of the circumstances and it is an object of the present invention to provide an electronic endoscope in which an insertion section can be reduced in a diameter because an image pickup apparatus can be further reduced in size and a distal end portion of the insertion section can be further reduced in size than in the past. Further, the present invention can also provide an image pickup apparatus and an electronic endoscope that can reduce, with simple configurations, noise that occurs in an image signal and obtain a stable image.

### Disclosure of Invention

### Means for Solving the Problem

An image pickup apparatus according to an aspect of the present invention includes: a solid-state image pickup device including a light-receiving element section that detects photographing light of a subject; a laminated substrate on which a plurality of electronic components configuring a driving circuit of the solid-state image pickup device are mounted and in which a plurality of conductor layers and a plurality of vias are formed; an electronic component connection land provided on a surface of the laminated substrate, any one of the plurality of electronic components being electrically connected to the electronic component connection land; and a cable connection land provided on the surface of the laminated substrate, a plurality of signal cables being electrically connected to the cable connection land. At least one of the plurality of electronic components is embedded in a position superimposed on the electronic component connection land or the cable connection land on an inside of the laminated substrate.

An image pickup apparatus according to another aspect of the present invention includes: a solid-state image pickup device including a light-receiving element section that detects a subject image; a circuit board section on which an electronic component configuring a driving circuit of the solid-state image pickup device is mounted and a step is provided; and a plurality of cables connected to a cable connection land provided in the circuit board section. Among the plurality of cables, an image cable for transmitting an image signal and a synchronization signal cable for transmitting a synchronization signal are connected to different surfaces of the circuit board section separated by the step.

An electronic endoscope according to an aspect of the present invention includes an image pickup apparatus provided at a distal end portion of an insertion section, the image pickup apparatus including: a solid-state image pickup device including a light-receiving element section that detects photographing light of a subject; a laminated substrate on which a plurality of electronic components configuring a driving circuit of the solid-state image pickup device are mounted and in which a plurality of conductor layers and a plurality of vias are formed; an electronic component connection land provided on a surface of the laminated substrate, any one of the plurality of electronic components being electrically connected to the electronic component connection land; and a cable connection land provided on the surface of the laminated substrate, a plurality of signal cables being electrically connected to the cable connection land. At least one of the plurality of electronic components is embedded in a position superimposed on the electronic component connection land or the cable connection land on an inside of the laminated substrate.

An electronic endoscope according to another aspect of the present invention includes an image pickup apparatus provided at a distal end portion of an insertion section, the image pickup apparatus including: a solid-state image pickup device including a light-receiving element section that detects a subject image; a circuit board section on which an electronic component configuring a driving circuit of the solid-state image pickup device is mounted and a step is provided; and a plurality of cables connected to a cable connection land provided in the circuit board section. Among the plurality of cables, an image cable for transmitting an image signal and a synchronization signal cable for transmitting a synchronization signal are connected to different surfaces of the circuit board section separated by the step.

According to the present invention described above, it is possible to provide an electronic endoscope in which an insertion section can be reduced in a diameter because an image pickup apparatus can be further reduced in size and a distal end portion of the insertion section can be further reduced in size than in the past. Further, in the invention of the other aspects, it is possible to obtain an image pickup apparatus and an electronic endoscope that can reduce, with simple configurations, noise that occurs in an image signal and obtain a stable image.

### Brief Description of the Drawings

Fig. 1 is a perspective view showing an exterior of an endoscope apparatus including an electronic endoscope in a first embodiment;
Fig. 2 is a plan view showing a configuration of a distal end face of a distal end portion in the first embodiment;
Fig. 3 is a plan view of a solid-state image pickup device and a laminated substrate viewed from a back in the first embodiment;
Fig. 4 is a top view showing a configuration of a circuit board of an image pickup apparatus in the first embodiment;
Fig. 5 is a sectional view showing a configuration of a distal end face of a sectional view distal end portion of a circuit board taken along line V-V in Fig. 4 in the first embodiment;
Fig. 6 is a sectional view showing an internal configuration of a distal end portion of an electronic endoscope according to a second embodiment;
Fig. 7 is a plan view of an image pickup apparatus viewed from a back in the second embodiment;
Fig. 8 is a sectional view showing a configuration of the image pickup apparatus taken along line VIII-VIII in Fig. 7 in the second embodiment;
Fig. 9 is a top view showing a configuration of the image pickup apparatus in the second embodiment;
Fig. 10 is a plan view of an image pickup apparatus of a modification of the second embodiment viewed from a back; and
Fig. 11 is a partial sectional view of a configuration of the image pickup apparatus taken along line VI-VI in Fig. 10 viewed from a side according to the modification of the second embodiment.

### Best Mode for Carrying Out the Invention

An endoscope including an image pickup apparatus in the present embodiment is explained with reference to the drawings.

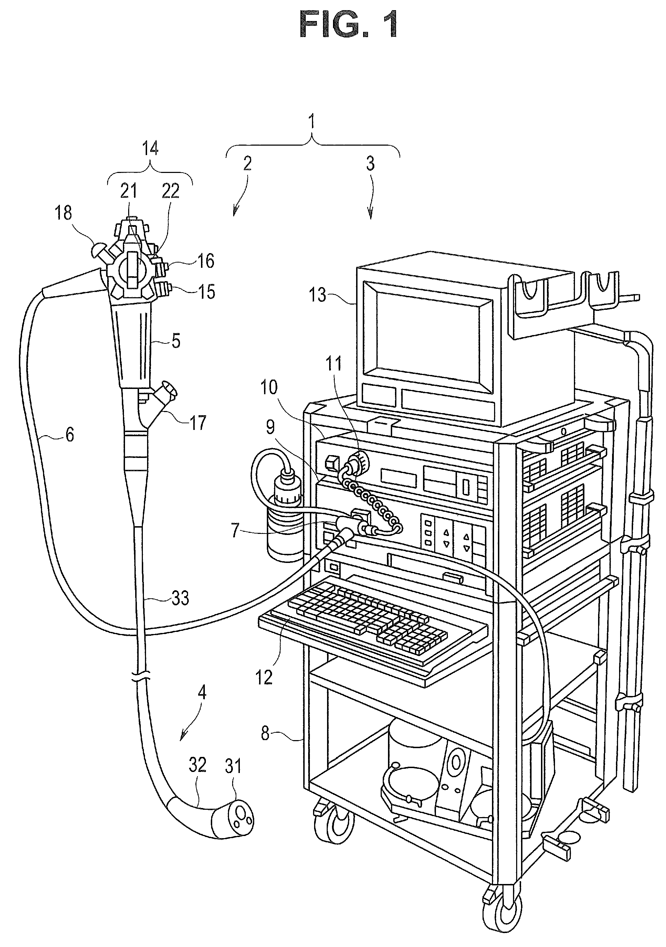

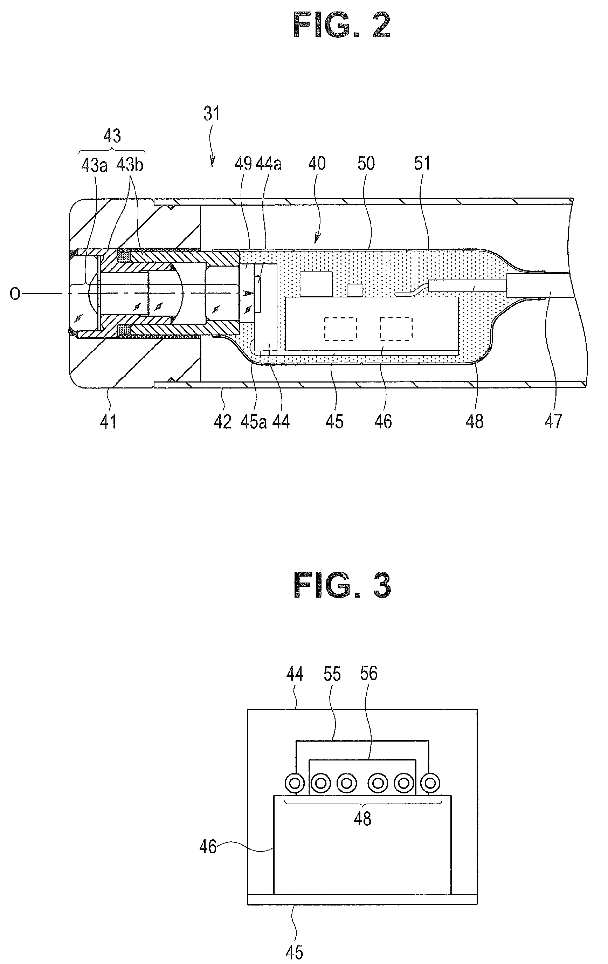

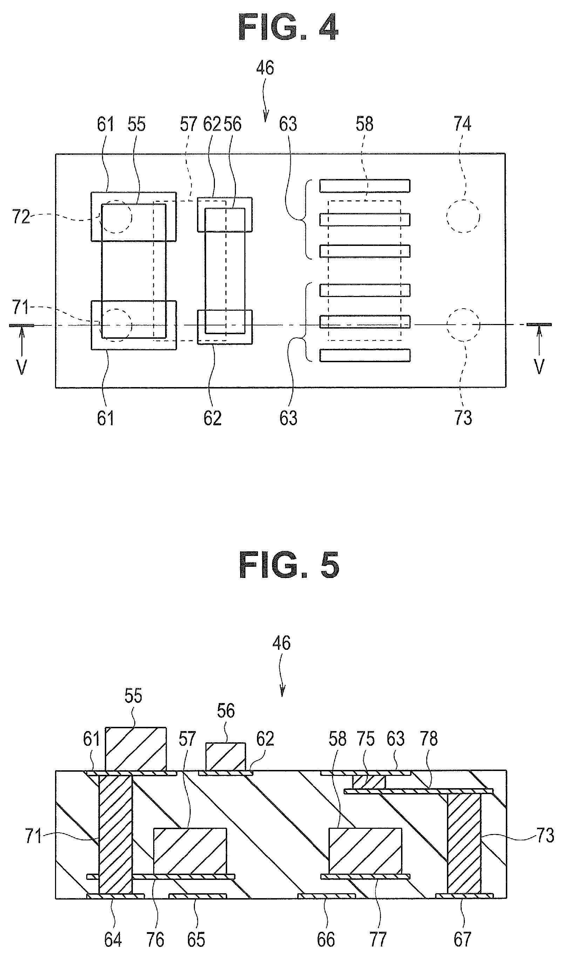

Fig. 1 is a perspective view showing an exterior of an endoscope apparatus including an electronic endoscope in the present embodiment. Fig. 2 is a plan view showing a configuration of a distal end face of a distal end portion. Fig. 3 is a plan view of a solid-state image pickup device and a laminated substrate viewed from a back. Fig. 4 is a top view showing a configuration of a circuit board of an image pickup apparatus. Fig. 5 is a sectional view of a circuit board taken along line V-V in Fig. 4.

Note that, in the following explanation, it should be noted that the drawings based on embodiments are schematic and relations between thicknesses and widths of respective portions, ratios of the thicknesses of the respective portions, and the like are different from real ones. Portions having different relations and ratios of dimensions are sometimes included among the drawings.

As shown in Fig. 1, a main part of an endoscope apparatus 1 is configured by an electronic endoscope (hereinafter simply referred to as endoscope) 2 and a peripheral apparatus 3. A main part of the endoscope 2 is configured from an insertion section 4 inserted into a subject, an operation section 5 connected to a proximal end side of the insertion section 4, a universal cord 6, and an endoscope connector 7.

In the peripheral apparatus 3, a system is configured by placing a light source apparatus 9, a video processor 10, a connection cable 11, a keyboard 12, a monitor 13, and the like on a stand 8. The endoscope 2 and the peripheral apparatus 3 having such configurations are connected to each other by the endoscope connector 7.

In the operation section 5 of the endoscope 2, a bending operation knob 14, buttons 15 and 16 for operating endoscope functions, a treatment instrument insertion port 17, and a turnable lever for fixing 18, which is an operation member, are provided. Note that the bending operation knob 14 is configured from a knob for up/down bending operation 21 and a knob for left/right bending operation 22.

The insertion section 4 of the endoscope 2 is configured by, in order from a distal end side, a distal end portion 31, a bending section 32 bendable in a plurality of directions, which is an operating section concatenated to a proximal end side of the distal end portion 31, and a flexible tube section 33 concatenated to a proximal end side of the bending section 32.

The bending section 32 operates with a turning operation inputted by the two knobs, i.e., the knob for up/down bending operation 21 and the knob for left/right bending operation 22 of the bending operation knob 14 provided in the operation section 5. That is, the bending section 32 bends according to operation of the bending operation knob 14 and is bendable in, for example, four directions of upward, downward, left, and right directions according to pulling and loosening of a bending operation wire (not shown in the figure) inserted through the insertion section 4.

The endoscope connector 7 provided at a distal end of the universal cord 6 of the endoscope 2 is connected to the light source apparatus 9 of the peripheral apparatus 3. In the endoscope connector 7, not-shown various caps and various electric contacts are provided. The endoscope connector 7 is electrically connected to the video processor 10 via the connection cable 11.

In the endoscope 2, a light guide bundle (not shown in the figure) for transmitting illumination light from the light source apparatus 9 is disposed. An illumination lens (not shown in the figure) is disposed at an emitting end of the illumination light emitted by the light guide bundle. The illumination lens is provided at the distal end portion 31 of the insertion section 4. The illumination light is irradiated to the subject.

Note that the configuration of the endoscope apparatus 1 explained above is only an example. The endoscope apparatus 1 is not limited to the configuration explained above.

Next, a configuration of the distal end portion 31 of the endoscope 2 in the present embodiment and a configuration of an image pickup apparatus disposed at the distal end portion 31 are explained in detail below.

As shown in Fig. 2, an image pickup apparatus 40 is provided inside the distal end portion 31 of the insertion section 4 of the endoscope 2. The image pickup apparatus 40 includes a substantially columnar distal end portion main body 41 that fits in and holds the image pickup apparatus 40. A rigid tube 42 forming an internal space for housing the image pickup apparatus in the distal end portion 31 is fit in a proximal end outer circumferential section of the distal end portion main body 41.

The image pickup apparatus 40 mainly includes a lens unit 43, a solid-state image pickup device 44, an FPC (flexible printed board) 45, and a laminated substrate 46, which is a circuit board. Note that a plurality of signal cables 48 of an electric cable bundle 47 are connected to the laminated substrate 46.

The lens unit 43 includes a lens holder 43b that holds a plurality of objective lenses 43a, which are objective optical systems. The lens holder 43b is inserted into and fit and fixed to the distal end portion main body 41. Consequently, the lens unit 43 is fixed to the distal end portion main body 41.

The solid-state image pickup device 44 is a CCD, a CMOS, or the like and includes a light-receiving element section 44a functioning as a pixel section that detects a subject image (photographing light indicated by a photographing optical axis O in the figure) focused by the plurality of objective lenses of the lens unit 43. A glass lid 49 is stuck to the solid-state image pickup device 44 to cover the light-receiving element section 44a.

A front surface of the glass lid 49 is firmly attached to the objective lens 43a at a most proximal end of the lens unit 43. That is, the lens unit 43 and the solid-state image pickup device 44 are firmly attached via the glass lid 49.

A flying lead (not shown in the figure) of the FPC 45 is electrically connected to an electrode (not shown in the figure) in a lower part of a surface of the solid-state image pickup device 44. The solid-state image pickup device 44 is sealed by sealing resin 45a. The sealing resin 45a covers and seals the flying lead bent at approximately 90° at a distal end of the FPC 45.

The FPC 45 is extended to a rear side from the solid-state image pickup device 44. The laminated substrate 46 is electrically and mechanically connected on the FPC 45. A circuit board section of the image pickup apparatus 40 is configured by the FPC 45 and the laminated substrate 46.

In the image pickup apparatus 40, a thermal contraction tube 50 is disposed to cover a proximal end portion of the lens unit 43 to a distal end portion of the electric cable bundle 47. In the thermal contraction tube 50, gaps among components are filled by filler resin 51. Note that the filler resin 51 may also be filled between the thermal contraction tube 50 and the rigid tube 42 to fill an internal space of the distal end portion 31.

The electric cable bundle 47 is disposed to be inserted through the insertion section 4 and is extended to the endoscope connector 7 via the operation section 5 and the universal cord 6 shown in Fig. 1.

The light guide bundle (not shown in the figure) described above is configured by bundling a plurality of optical fibers for transmitting the illumination light from the light source apparatus 9 to the distal end portion. The light guide bundle is disposed to be inserted through the bending section 32 and the flexible tube section 33 of the insertion section 4 in a state in which the light guide bundle is coated with an outer coat. The light guide bundle is extended to the endoscope connector 7 via the operation section 5 and the universal cord 6 shown in Fig. 1.

Next, a configuration of the laminated substrate 46 in the present embodiment is explained below.

On the laminated substrate 46, as shown in Fig. 3 to Fig. 5, at least one, here, two electronic components 55 and 56 are mounted on an upper part surface (an upper surface) and at least one, here, two electronic components 57 and 58 are embedded in an inside. Four electronic components 55, 56, 57, and 58 in total configuring a driving circuit of the solid-state image pickup device 44 explained below are provided.

On the upper part surface of the laminated substrate 46, as shown in Fig. 4 and Fig. 5, two electronic component connection lands 61 to which a first electronic component 55 is electrically connected, two electronic component connection lands 62 to which a second electronic component 56 is electrically connected, and six cable connection lands 63 to which a plurality of, here, six signal cables 48 are electrically connected by solder or the like are formed.

On a lower part surface of the laminated substrate 46, a plurality of substrate connection lands 64, 65, 66, and 67 electrically connected to connection terminals of the FPC 45 are formed.

On an inside of the laminated substrate 46, a plurality of conductor layers 76, 77, and 78 are stacked. The conductor layers 76, 77, and 78 are formed to be electrically connected to any ones of a plurality of vias 71, 72, 73, 74, and 75 provided inside the laminated substrate 46.

For example, the two electronic component connection lands 61 on the upper surface side of the laminated substrate 46 are electrically connected to the different two substrate connection lands 64 (in Fig. 5, only one is shown) on a lower surface side of the laminated substrate 46 via the vias 71 and 72. The two electronic component connection lands 62 on the upper surface side of the laminated substrate 46 are electrically connected to the different two substrate connection lands 67 (in Fig. 5, only one is shown) on the lower surface side of the laminated substrate 46 via the vias 73 and 74.

Note that the third electronic component 57 is electrically connected to the conductor layer 76. The fourth electronic component 58 is electrically connected to the conductor layer 77. The conductor layers 76 and 77 are electrically connected to, via not-shown vias, the substrate connection lands 65 and 66 formed on the lower surface side of the laminated substrate 46.

The six cable connection lands 63 are respectively individually electrically connected to the conductor layers 76, 77, and 78 and the like via the plurality of vias 75 (in Fig. 5, only one is shown). Note that the conductor layer 78 is electrically connected to the substrate connection lands 67 on the lower surface side via the vias 73 (or the vias 74) (in Fig. 5, only one substrate connection land 67 and only one via 73 are shown).

In the laminated substrate 46 in the present embodiment, the third electronic component 57 is embedded in a position where the third electronic component 57 overlaps, in top view, the plurality of electronic component connection lands 61 and 62 formed on the surface on the upper part side to which the first electronic component 55 or the second electronic component 56 are connected. The fourth electronic component 58 is embedded in a position where the fourth electronic component 58 overlaps, in top view, the plurality of cable connection lands 63 formed on the surface on the upper part side to which the signal cables 48 are connected.

In other words, in the laminated substrate 46, the third electronic component 57 is embedded in a position where the third electronic component 57 is superimposed on the plurality of electronic component connection lands 61 and 62 to which the first electronic component 55 or the second electronic component 56 is connected. The fourth electronic component 58 is embedded in a position where the fourth electronic component 58 is superimposed on the plurality of cable connection lands 63 connected to the signal cables 48.

Consequently, for example, compared with a configuration in which all the electronic components 55, 56, 57, and 58 are mounted and the plurality of cable connection lands 63 to which the signal cables 48 are connected are disposed over the entire surface on the upper part side, in the laminated substrate 46, it is possible to reduce a substrate area in top view. As a result, it is possible to reduce size of the image pickup apparatus 40.

In addition, as shown in Fig. 3, the entire laminated substrate 46 in the present embodiment including the first electronic component 55 and the second electronic component 56 mounted on the upper surface and the plurality of signal cables 48 connected to the upper surface is disposed to be fit within a projection area of the solid-state image pickup device 44. That is, the entire laminated substrate 46 is disposed to be fit within the projection area of the solid-state image pickup device 44 with respect to a direction along the photographing optical axis O. Therefore, it is possible to realize a reduction in the size of the image pickup apparatus 40.

Incidentally, when the plurality of conductor layers 76, 77, and 78 are stacked and formed in parallel to a plane on which the light-receiving element section 44a is provided on the laminated substrate 46, the laminated substrate 46 is increased in size in a height direction. It is difficult to fit the laminated substrate 46 within the projection area of the solid-state image pickup device 44.

Therefore, in the laminated substrate 46 in the present embodiment, the plurality of conductor layers 76, 77, and 78 on the inside are stacked and formed to extend in a direction perpendicular to the plane on which the light-receiving element section 44a of the solid-state image pickup device 44 is provided.

That is, the plurality of conductor layers 76, 77, and 78 are stacked and formed to extend in a direction orthogonal to the plane of the solid-state image pickup device 44 on which the light-receiving element section 44a is provided. Consequently, it is possible to reduce height of the laminated substrate 46. It is possible to fit the laminated substrate 46 within the projection area of the solid-state image pickup device 44.

Further, in a manufacturing process for the laminated substrate 46, it is necessary to provide a predetermined distance between the third electronic component 57 and the fourth electronic component 58 to be embedded and the plurality of vias 71, 72, 73, and 74 and separate the third electronic component 57 and the fourth electronic component 58 and the plurality of vias 71, 72, 73, and 74 from each other.

Therefore, in the present embodiment, the plurality of vias 71, 72, 73, and 74 are provided adjacent to the third electronic component 57 and the fourth electronic component 58 to be embedded in a front-back direction of the laminated substrate 46 and are provided in parallel along a longitudinal direction of the laminated substrate 46 to prevent expansion of the laminated substrate 46 in a width direction and reduce width of the laminated substrate 46. As a result, it is possible to reduce a diameter of the image pickup apparatus 40.

### (Second Embodiment)

An endoscope including an endoscope distal end structure in a second embodiment is explained below with reference to the drawings.

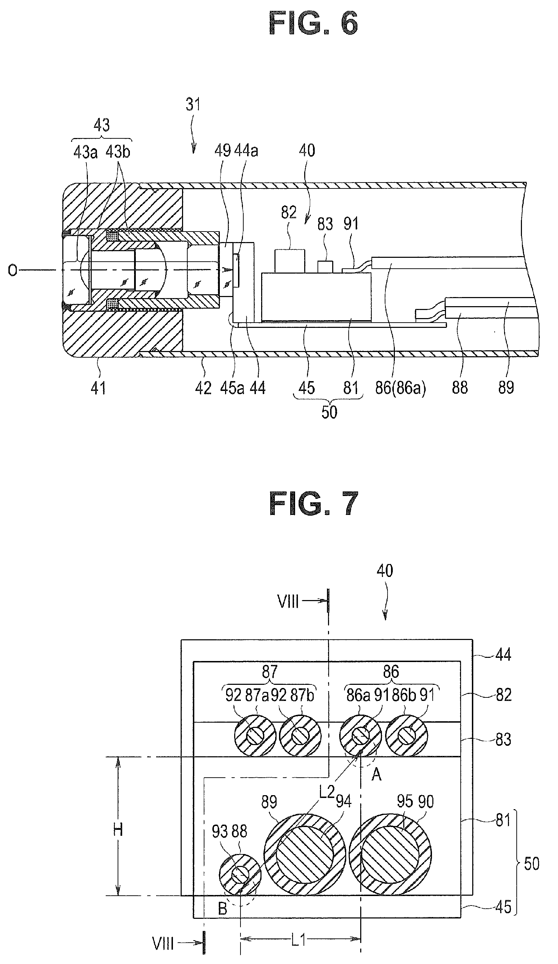

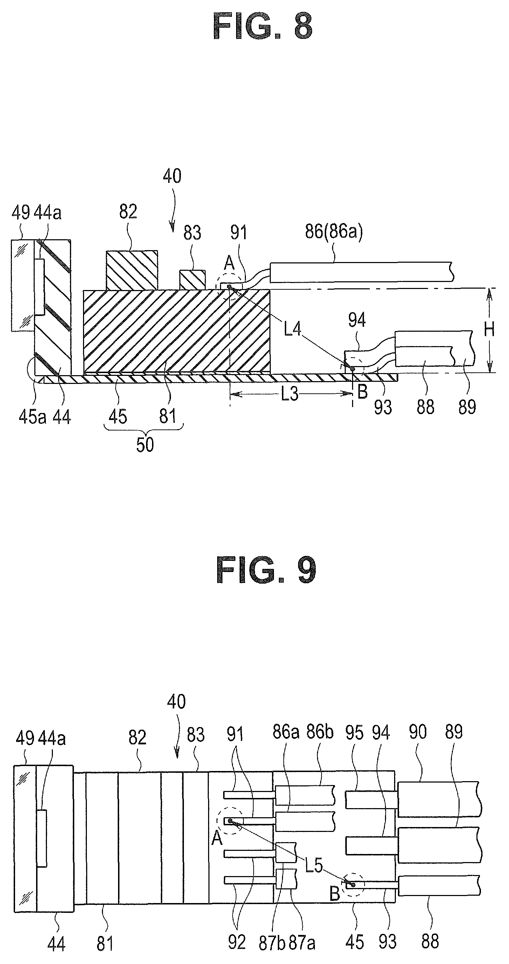

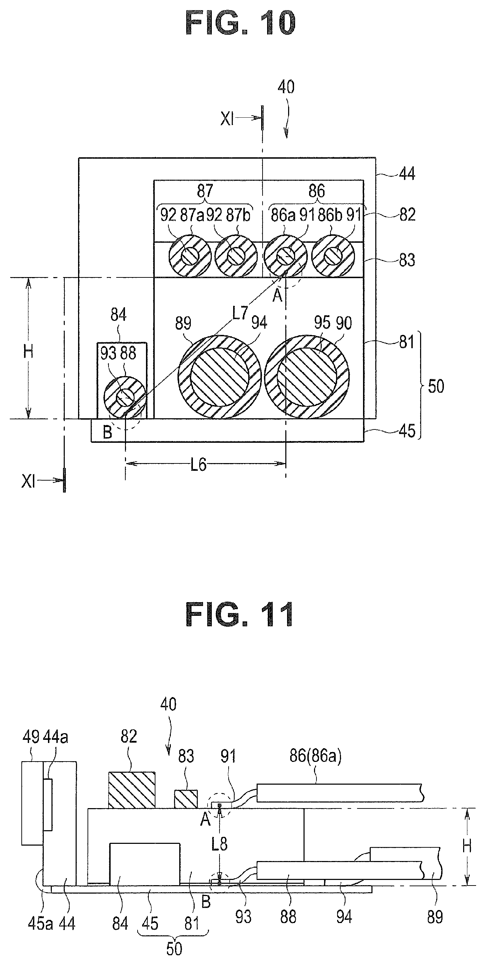

Fig. 6 is a sectional view showing an internal configuration of a distal end portion of an electronic endoscope. Fig. 7 is a plan view of an image pickup apparatus viewed from a back. Fig. 8 is a sectional view showing a configuration of the image pickup apparatus taken along line VIII-VIII in Fig. 7. Fig. 9 is a top view showing a configuration of the image pickup apparatus. Fig. 10 is a plan view of an image pickup apparatus of a modification viewed from a back. Fig. 11 is a partial sectional view of a configuration of the image pickup apparatus taken along line VI-VI in Fig. 10 viewed from a side.

Note that, in the following explanation, components common to the various components in the first embodiment explained above are denoted by the same reference numerals and signs. Detailed explanation of the components is omitted.

First, a configuration of the distal end portion 31 of the endoscope 2 in the present embodiment and a configuration of an image pickup apparatus disposed at the distal end portion 31 are explained in detail below.

As shown in Fig. 6, as in the first embodiment, the image pickup apparatus 40 is provided inside the distal end portion 31 of the insertion section 4 of the endoscope 2.

The image pickup apparatus 40 mainly includes the lens unit 43, the solid-state image pickup device 44, the FPC (flexible printed board) 45, and a rigid substrate 81 functioning as a circuit board. Note that a plurality of various cables 86, 87, and 88, a power supply cable 89, or a ground (GND) cable 90 are connected to the rigid substrate 81 and the FPC 45 (see Fig. 7).

The rigid substrate 81 is electrically and mechanically connected to be stacked on a surface on an upper part side on a front side of the FPC 45 provided in the image pickup apparatus 40.

A circuit board section 85 of the image pickup apparatus 40 is configured by the FPC 45 and the rigid substrate 81. Note that the FPC 45 has length extending further backward than a proximal end of the rigid substrate 81.

That is, a dimension in a longitudinal direction of a base material section configured from a base film, a conductor, a cover lay, and the like of the FPC 45 is set larger than a dimension in a longitudinal direction of the rigid substrate 81. Note that the longitudinal direction of the FPC 45 is a direction along the photographing optical axis O.

A plurality of cable connection lands (not shown in the figure) are formed on a surface of the base material section of the FPC 45 extended backward. The synchronization signal cable 88, the power supply cable 89, and the GND cable 90 are connected to the plurality of cable connection lands.

That is, in the FPC 45, a plurality of cable connection lands (not shown in the figure) are provided on the base material section exposed backward from the rigid substrate 81 in a state in which the rigid substrate 81 is stacked. The synchronization signal cable 88, the power supply cable 89, and the GND cable 90 are soldered to the plurality of cable connection lands.

The rigid substrate 81 is a substrate formed of a glass epoxy substrate, a ceramic substrate, or the like. A plurality of, here, two electronic components 82 and 83 configuring a circuit for driving the solid-state image pickup device are mounted on an upper surface of the rigid substrate 81.

A plurality of cable connection lands (not shown in the figure) are formed on a surface in a back of the rigid substrate 81. The image cable 86 and the other control cable 87 are soldered and connected to the plurality of cable connection lands (not shown in the figure).

Note that the rigid substrate 81 may be configured in a small size obtained by reducing a substrate area using a component-incorporated substrate in which electronic components are incorporated.

In this way, in the image pickup apparatus 40 in the present embodiment, steps provided with level differences are formed on a substrate surface on an upper part side of the FPC 45 and a substrate surface on an upper part side of the rigid substrate 81 configuring the circuit board section 85.

The synchronization signal cable 88, the power supply cable 89, and the GND cable 90 are connected to a substrate surface of the FPC 45. The image cable 86 and the other control cable 87 are connected to a substrate surface of the rigid substrate 81.

The image cable 86, the other control cable 87, the synchronization signal cable 88, the power supply cable 89, and the GND cable 90 are bound from a proximal end portion of the distal end portion 31 and covered with an outer coat sheath to be disposed to be inserted through the insertion section 4 as a not-shown electric cable bundle. The electric cable bundle is extended to the endoscope connector 7 via the operation section 5 and the universal cord 6 shown in Fig. 1.

Note that, in the image pickup apparatus 40, a thermal contraction tube may be provided to cover a proximal end portion of the connected lens unit 43 to a distal end portion of the electric cable bundle. In the thermal contraction tube, sealing resin for filling gaps among components is desirably filled. Further, sealing resin may also be filled between the thermal contraction tube and the rigid tube 42 to fill an internal space of the distal end portion 31.

The light guide bundle (not shown in the figure) described above is configured by bundling a plurality of optical fibers for transmitting the illumination light from the light source apparatus 9 to the distal end portion. The light guide bundle is disposed to be inserted through the bending section 32 and the flexible tube section 33 of the insertion section 4 in a state in which the light guide bundle is coated with an outer coat. The light guide bundle is extended to the endoscope connector 7 via the operation section 5 and the universal cord 6 shown in Fig. 1.

Next, a configuration for connecting the image cable 86, the other control cable 87, the synchronization signal cable 88, the power supply cable 89, and the GND cable 90 to the rigid substrate 81 in the image pickup apparatus 40 in the present embodiment is explained in detail below.

As shown in Fig. 7, the image cable 86 including a pair of image signal cables 86a and 86b and the other control cable 87 including a pair of control signal cables 87a and 87b are provided in parallel in a width direction of the rigid substrate 81. Respective cable core wires 91 and 92, from which outer coats of the cables are stripped off, are connected to, by soldering, a plurality of cable connection lands (not shown in the figure) provided on a surface of the rigid substrate 81.

Specifically, when the image pickup apparatus 40 is viewed from a back, as shown in Fig. 7, the pair of image signal cables 86a and 86b and the pair of control signal cables 87a and 87b are connected to be provided in parallel on the surface of the rigid substrate 81 on an upper stage side of the circuit board section 85 such that the pair of image signal cables 86a and 86b is on a right side and the pair of control signal cables 87a and 87b is on a left side.

The synchronization signal cable 88, the power supply cable 89, and the GND cable 90 are provided in parallel in a width direction of the FPC 45. Cable core wires 93, 94, and 95, from which outer coats of the cables are removed, are connected to, by soldering, a plurality of cable connection lands (not shown in the figure) provided on a surface of the FPC 45 on a lower stage side of the circuit board section 85.

That is, the image cable 86 and the other control cable 87 and the synchronization signal cable 88, the power supply cable 89, and the GND cable 90 are connected to be separated a predetermined distance H in a height direction of the circuit board section 85, which is a level difference between the surface of the FPC 45 and the surface of the rigid substrate 81.

Note that the image cable 86 is connected in a position where both of one image signal cable 86a connected closer to a center on an inner side of the rigid substrate 81 and the other image signal cable 86b connected closer to a side (a right side viewed from the back) on an outer side of the rigid substrate 81 are separated the predetermined distance H in the height direction of the circuit board section 85 with respect to the synchronization signal cable 88.

Therefore, the image cable 86 and the synchronization signal cable 88 are connected in positions separated at least the predetermined distance H in the height direction of circuit board section 85.

In addition, a connecting section A where the cable core wire 91 of the one image signal cable 86a of the image cable 86 is connected to the cable connection land and a connecting section B where the cable core wire 93 of the synchronization signal cable 88 is connected to the cable connection land on a left side of the FPC 45 viewed from the back are connected to be separated a predetermined distance L1 in a width direction of the circuit board section 85.

Therefore, the other image signal cable 86b located closer to a side (in Fig. 7, a right side) of the rigid substrate 81 than the one image signal cable 86a is also connected in a position separated the predetermined distance L1 or more in the width direction of the circuit board section 85 with respect to the synchronization signal cable 88.

Therefore, the image cable 86 and the synchronization signal cable 88 are connected in positions separated at least the predetermined distance L1 in the width direction of the circuit board section 85.

Note that the connecting section A of the image signal cable 86a and the connecting section B of the synchronization signal cable 88 are separated a predetermined distance L2 in a diagonal direction in a cross section orthogonal to the longitudinal direction (a cross section in a latitudinal direction) of the FPC 45 and the rigid substrate 81 configuring the circuit board section 85 of the image pickup apparatus 40. Note that the longitudinal direction of the circuit board section 85 is a direction along the photographing optical axis O.

Therefore, the other image signal cable 86b located closer to the side of the rigid substrate 81 than the one image signal cable 86a is also connected in a position separated the predetermined distance L2 or more in the diagonal direction in the cross section orthogonal to the longitudinal direction (the cross section in the latitudinal direction) of the circuit board section 85 with respect to the synchronization signal cable 88.

Therefore, the image cable 86 and the image signal cable 86b are connected in positions separated at least the predetermined distance L2 in the diagonal direction in the cross section orthogonal to the longitudinal direction (the cross section in the latitudinal direction) of the circuit board section 85.

Further, as shown in Fig. 8, the connecting section A of the image signal cable 86a and the connecting section B of the synchronization signal cable 88 are connected in positions separated a predetermined distance L3 in the longitudinal direction in side view of the circuit board section 85.

Therefore, the other image signal cable 86b provided in parallel to the one image signal cable 86a in the width direction is also connected in a position separated the predetermined distance L3 or more in the longitudinal direction in side view of the circuit board section 85 with respect to the synchronization signal cable 88.

Therefore, the image cable 86 and the image signal cable 86b are connected in positions separated at least the predetermined distance L3 in the longitudinal direction in side view of the circuit board section 85.

Note that the connecting section A of the image signal cable 86a and the connecting section B of the synchronization signal cable 88 are separated a predetermined direction L4 in the diagonal direction in the cross section in the longitudinal direction of the FPC 45 and the rigid substrate 81 configuring the circuit board section 85 of the image pickup apparatus 40.

Therefore, the other image signal cable 86b provided in parallel to the one image signal cable 86a is also connected in a position separated the predetermined distance L4 or more in the diagonal direction in the cross section in the longitudinal direction of the circuit board section 85 with respect to the synchronization signal cable 88.

Therefore, the image cable 86 and the image signal cable 86b are connected to be separated at least the predetermined distance L4 in the diagonal direction in the cross section in the longitudinal direction of the circuit board section 85.

Further, as shown in Fig. 9, the connecting section A of the image signal cable 86a and the connecting section B of the synchronization signal cable 88 are connected in positions separated a predetermined distance L5 in top view of the circuit board section 85.

Therefore, the other image signal cable 86b provided in parallel to the one image signal cable 86a in the width direction is also connected in a position separated the predetermined distance L5 or more in top view of the circuit board section 85 with respect to the synchronization signal cable 88.

Therefore, the image cable 86 and the image signal cable 86b are connected in positions separated at least the predetermined distance L5 in top view of the circuit board section 85.

As explained above, the image pickup apparatus 40 in the present embodiment has a configuration in which, in particular, the step of the predetermined distance H is provided between the surface of the FPC 45 to which the synchronization signal cable 88 is connected and the surface of the rigid substrate 81 to which the image cable 86 is connected and, in addition to the level difference by the step, the synchronization signal cable 88 and the image cable 86 are connected in the positions separated the predetermined distance L3 in side view in the longitudinal direction of the circuit board section 85 and the positions separated the predetermined distance L5 in top view.

In this way, the image pickup apparatus 40 has a configuration in which the image cable 86 and the synchronization signal cable 88 are spatially separated and connected. Therefore, it is possible to suppress influence of an electromagnetic wave from the synchronization signal cable 88 to the image cable 86 and suppress noise caused by interference of the electromagnetic wave with an image signal transmitted by the image cable 86. Consequently, an endoscopic image displayed on the monitor is stable without being disturbed.

Note that the configuration of the image pickup apparatus 40 is explained in which the step of the predetermined distance H is provided in the height direction according to the thickness of the rigid substrate 81, a bonding layer with the FPC 45, and the like. The level difference of the step is desirably within a range of 0.2 mm to 4.0 mm. A size of the step can be selected according to a height dimension of the solid-state image pickup device 44 and thicknesses of the various cables.

Further, the rigid substrate 81, the electronic components 82 and 83 mounted on the rigid substrate 81, and various cables including the image cable 86, and the other control cable 87, the synchronization signal cable 88, the power supply cable 89, and the GND cable 90 are disposed to be fit within the projection area of the solid-state image pickup device 44. Consequently, it is possible to realize the small image pickup apparatus 40.

That is, in the image pickup apparatus 40, as shown in Fig. 7, all of the rigid substrate 81, the two electronic components 82 and 83 mounted on the upper surface of the rigid substrate 81, and the various cables including the image cable 86, the other control cable 87, the synchronization signal cable 88, the power supply cable 89, and the GND cable 90 connected to the FPC 45 or the rigid substrate 81 are fit within the projection area of the solid-state image pickup device 44. Therefore, it is possible to realize a reduction in the size of the image pickup apparatus 40.

Note that, in the image pickup apparatus 40, it is desirable that the rigid substrate 81, the electronic component 82 and 83, and all of the various cables are fit within the projection area of the solid-state image pickup device 44 and the step is formed by the surface of the FPC 45 and the surface of the rigid substrate 81 having the predetermined distance H to prevent the synchronization signal cable 88, the power supply cable 89, and the GND cable 90 connected to the cable connection land on the lower stage side provided in the FPC 45 from interfering with the image cable 86 and the other control cable 87 connected to the cable connection land on the upper stage side provided on the rigid substrate 81.

The level difference of such a step can be easily obtained as a relatively large level difference having the predetermined distance H according to the thickness of the rigid substrate 81. Therefore, the power supply cable 89, the GND cable 90, and the like having relatively large diameters are connected to the cable connection land on the lower stage side provided on the FPC 45. Consequently, the power supply cable 89 and the GND cable 90 less easily protrude from the projection area of the solid-state image pickup device 44. The image pickup apparatus 40 is easily reduced in a diameter.

Note that, among the various cables, coaxial cables are used as the cables having the relatively large diameters. Therefore, not only the power supply cable 89 or the GND cable 90 but also the coaxial cables are desirably connected to the cable connection land on the lower stage side provided on the FPC 45 to prevent the cables from easily protruding from the projection area of the solid-state image pickup device 44.

Further, the image pickup apparatus 40 explained above has the configuration in which the image cable 86 is connected to the upper stage side of the circuit board section 85 and the synchronization signal cable 88 is connected to the lower stage side of the circuit board section 85. However, since the synchronization signal cable 88 only has to be spatially separated from the image cable 86, the image pickup apparatus 40 may have a configuration in which the image cable 86 is connected to the lower stage side and the synchronization signal cable 88 is connected to the upper stage side respectively in positions separated the predetermined distance (L1 to L5) described above.

According to the above explanation, the image pickup apparatus 40 in the present embodiment can be configured in a simple configuration, in particular, without providing a relay member on the cable connection land of the rigid substrate 81, providing a level difference in the connecting sections of the various signal cables, and providing lands for cable connection on front and rear surfaces of the FPC 45 or the rigid substrate 81 and can be configured in small size in which noise that occurs in an image signal is reduced and a stable image is obtained. In this way, since the image pickup apparatus 40 is reduced in size, in the endoscope 2, it is possible to reduce size of the distal end portion 31 of the insertion section 4 in which the image pickup apparatus 40 is mounted. As a result, it is possible to reduce the insertion section 4 in a diameter.

### (Modification)

Note that, as shown in Fig. 10 and Fig. 11, it is also possible that a dimension in the width direction of the rigid substrate 81 is reduced, an empty region is provided from a side surface of the rigid substrate 81 to the FPC 45, and the electronic component 84 is mounted or the synchronization signal cable 88 is connected on the region.

In this modification, as in the above explanation, the connecting section A where the cable core wire 91 of the one image signal cable 86a of the image cable 86 is connected to the cable connection land and the connecting section B where the cable core wire 93 of the synchronization signal cable 88 is connected to the cable connection land on the left side of the FPC 45 viewed from the back are connected to be separated a predetermined direction L6 in the width direction of the circuit board section 85 as shown in Fig. 10.

Therefore, the other image signal cable 86b located closer to the side of the rigid substrate 81 than the one image signal cable 86a is also connected in a position separated the predetermined distance L6 or more in the width direction of the circuit board section 85 with respect to the synchronization signal cable 88.

Therefore, the image cable 86 and the synchronization signal cable 88 are connected in positions separated at least the predetermined distance L6 in the width direction of the circuit board section 85.

Further, the connecting section A of the image signal cable 86a and the connecting section B of the synchronization signal cable 88 are separated a predetermined distance L7 in the diagonal direction in the cross section orthogonal to the longitudinal direction of the circuit board section 85.

Therefore, the other image signal cable 86b located closer to the side of the rigid substrate 81 than the one image signal cable 86a is also connected in a position separated the predetermined distance L7 or more in the diagonal direction in the cross section orthogonal to the longitudinal direction of the circuit board section 85 with respect to the synchronization signal cable 88.

Therefore, the image cable 86 and the image signal cable 86b are connected in positions separated at least the predetermined distance L7 in the diagonal direction in the cross section orthogonal to the longitudinal direction (the cross section in the latitudinal direction) of the circuit board section 85.

Note that, in the longitudinal direction of the circuit board section 85, as shown in Fig. 11, the connecting section A of the image signal cable 86a and the connecting section B of the synchronization signal cable 88 are connected in the same position and separated a predetermined distance L8 in the height direction of the circuit board section 85.

Therefore, the other image signal cable 86b located closer to the side of the rigid substrate 81 than the one image signal cable 86a is also connected in a position separated the predetermined distance L8 in the height direction of the circuit board section 85 with respect to the synchronization signal cable 88.

Therefore, the image cable 86 and the image signal cable 86b are connected in positions separated at least the predetermined distance L8 in the height direction of the rigid substrate 81. The predetermined distance L8 is substantially the same as the predetermined distance H in the height direction of the image pickup apparatus 40, which is the level difference by the surface of the FPC 45 and the surface of the rigid substrate 81 (L8 ≈ H).

With such a configuration as well, in the image pickup apparatus 40, the image cable 86 and the synchronization signal cable 88 can be spatially separated and connected. Although an effect is weaker than the effect of the configuration explained above, it is possible to suppress influence of an electromagnetic wave from the synchronization signal cable 88 to the image cable 86 and suppress noise caused by interference of the electromagnetic wave with an image signal transmitted by the image cable 86. As a result, an endoscopic image displayed on the monitor is stable without being disturbed.

The invention described in the embodiments described above is not limited to the embodiments and the modifications. Besides, in an implementation stage, various variations can be carried out in a range not departing from the spirit of the invention. Further, the embodiments include inventions in various stages. Various inventions can be extracted according to appropriate combinations of a disclosed plurality of constituent elements.

For example, when the described problems can be solved and the described effects can be obtained even if several constituent elements are deleted from all the constituent elements described in the embodiments, a configuration in which the constituent elements are deleted can be extracted as an invention.

This application is based upon and claims the benefit of priority from Japanese Patent Application No. 2013-087583 and Japanese Patent Application No. 2013-087584 filed in Japan on April 18, 2013, the contents described above are cited in the specifications, the claims, and the drawings of Japanese Patent Application No. 2013-087583 and Japanese Patent Application No. 2013-087584.

## Claims

1. An image pickup apparatus comprising:
a solid-state image pickup device including a light-receiving element section that detects a subject image;
a laminated substrate on which a plurality of electronic components configuring a driving circuit of the solid-state image pickup device are mounted and in which a plurality of conductor layers and a plurality of vias are formed;
an electronic component connection land provided on a surface of the laminated substrate, any one of the plurality of electronic components being electrically connected to the electronic component connection land; and
a cable connection land provided on the surface of the laminated substrate, a plurality of signal cables being electrically connected to the cable connection land, wherein
at least one of the plurality of electronic components is embedded in a position superimposed on the electronic component connection land or the cable connection land on an inside of the laminated substrate.

2. The image pickup apparatus according to claim 1, wherein the laminated substrate, the plurality of electronic components, and the plurality of signal cables are disposed to be fit within a projection plane of the solid-state image pickup device with respect to a direction along a photographing optical axis of the subject image.

3. The image pickup apparatus according to claim 1 or 2, wherein the conductor layer of the laminated substrate is stacked and formed to extend in a direction orthogonal to a plane on which a light-receiving element section of the solid-state image pickup device is formed.

4. The image pickup apparatus according to any one of claims 1 to 3, wherein at least one of the electronic components and at least one of the vias embedded in the laminated substrate are provided adjacent to each other and provided in parallel along a longitudinal direction of the stacked substrate.

5. An image pickup apparatus comprising:
a solid-state image pickup device including a light-receiving element section that detects a subject image;
a circuit board section on which an electronic component configuring a driving circuit of the solid-state image pickup device is mounted and a step is provided; and
a plurality of cables connected to a cable connection land provided in the circuit board section, wherein
among the plurality of cables, an image cable for transmitting an image signal and a synchronization signal cable for transmitting a synchronization signal are connected to different surfaces of the circuit board section separated by the step.

6. The image pickup apparatus according to claim 5, wherein
in the circuit board section, a rigid substrate is stacked and connected to be formed on a flexible printed board and the step separated in a height direction is formed by a surface of the flexible printed board and a surface of the rigid substrate, and
the image cable and the synchronization signal cable are connected in positions separated from each other in the height direction of the circuit board section.

7. The image pickup apparatus according to claim 5 or 6, wherein the image cable and the synchronization signal cable are connected in positions separated from each other in a longitudinal direction along a photographing optical axis of the subject image of the circuit board section.

8. The image pickup apparatus according to any one of claims 5 to 7, wherein the image cable and the synchronization signal cable are connected in positions separated in a width direction of the circuit board section.

9. The image pickup apparatus according to any one of claims 5 to 8, wherein the image cable and the synchronization signal cable are connected in positions separated in a diagonal direction in a cross section orthogonal to a longitudinal direction along the photographing optical axis of the subject image of the circuit board section.

10. The image pickup apparatus according to any one of claims 5 to 9, wherein the rigid substrate, the electronic component, and the plurality of cables are fit within a projection area of the solid-state image pickup device along the photographing optical axis of the subject image.

11. The image pickup apparatus according to any one of claims 6 to 9, wherein, among the plurality of cables, the power supply cable and/or the ground cable is connected to a lower stage side in the height direction of the step.

12. The image pickup apparatus according to any one of claims 5 to 11, wherein the step is within a range of 0.2 mm to 4.0 mm.

13. An electronic endoscope comprising the image pickup apparatus according to any one of claims 1 to 12 provided at a distal end portion of an insertion section.
